# FASCICULE DE BREVET EUROPEEN

(11) **EP 0 524 064 B1**
(45) Date de publication et mention de la délivrance du brevet: **15.03.1995**
(21) Numéro de dépôt: 92401983.9
(22) Date de dépôt: 09.07.1992
(51) Int. Cl.: A61B 6/00

(54) **Mobile radiologique**
Mobiles Röntgengerät
Mobile X-ray device

(30) Priorité: 19.07.1991 FR 9109186
(43) Date de publication de la demande: 20.01.1993
(73) Titulaire: GENERAL ELECTRIC CGR S.A., F-92130 Issy les Moulineaux (FR)
(72) Inventeur: Varisco, Piero, Cabinet BALLOT-SCHMIT, F-75116 Paris (FR)
(74) Mandataire: Schmit, Christian Norbert Marie

(56) Documents cités:
- FR-A- 2 175 841
- FR-A- 2 405 612
- FR-A- 2 405 695

## Description

La présente invention a pour objet un mobile radiologique utilisable principalement dans le domaine médical. Un mobile radiologique est un appareil radiologique portant un tube à rayons X et permettant d'approcher ce tube à rayons X à proximité d'un patient dans n'importe quelle position et sous n'importe quelle incidence. Un tel mobile permet notamment d'effectuer des radiographies sur des patients qu'il est par ailleurs impossible d'amener sur une table de radiologie classique parce que leur état de santé ne le permettrait pas.

On connaît des mobiles radiologiques constitués d'un chariot d'assise rectangulaire et supportant une structure de bras articulés au bout desquels est fixé le tube à rayons X. Au cours de l'utilisation, la structure des bras est déployée et le tube à rayons X est présenté en porte à faux par rapport à l'assise du chariot. Il peut ainsi être déplacé sur l'avant ou sur les côtés du chariot. Il existe des normes de stabilité auxquelles doivent souscrire ces mobiles radiologiques. Par exemple, l'application d'un effort de 23 kg sur le tube à rayons X, dans le but de renverser le chariot, doit être contrarié avec efficacité pour n'importe quelle position de ce tube. Pour satisfaire à ces contraintes, on a fabriqué des mobiles radiologiques dont le poids est très élevé. Par exemple, on connaît des mobiles radiologiques dont le poids total est de l'ordre de 450 Kg. Le déplacement de ces mobiles ne peut être effectué qu'au moyen de moteurs tant ils sont lourds.

Dans l'invention on a cherché à réduire le poids d'un tel mobile radiologique dans le but de pouvoir enlever, éventuellement, les moteurs tout en souscrivant par ailleurs à la norme anti-basculement.

Pour résoudre ces problèmes, dans l'invention, on a muni la structure des bras déployables d'un mécanisme de limitation du déploiement qui tient compte du caractère rectangulaire du chariot. En effet, lorsque le bras se déploie vers l'avant du chariot, étant donné que le chariot est long, le bras peut être étendu plus loin que lorsque ses bras s'étendent latéralement au chariot puisque le chariot est moins large qu'il n'est long. En conséquence, le couple de renversement vers l'avant doit être plus grand, pour un déploiement donné, que le couple de renversement sur le côté. Par conséquent, on peut autoriser, vers l'avant, un déploiement plus grand. En conséquence dans l'invention on a muni le mobile de moyens par lesquels le déploiement de la structure est fonction de l'angle d'orientation de ce déploiement. Vers l'avant le déploiement est maximal, sur les côtés il est limité.

L'invention a donc pour objet un mobile radiologique comportant un chariot mobile sur le sol, une structure déployable reliée mécaniquement au chariot par une première extrémité et à un tube à rayons X par sa deuxième extrémité, la structure pouvant être déployée en porte-à-faux par rapport au chariot et pouvant tourner autour d'un axe vertical, caractérisé en ce qu'il comporte des moyens pour que l'amplitude maximum du déploiement soit fonction de l'angle de rotation de cette structure autour de l'axe vertical.

L'invention sera mieux comprise à la lecture de la description qui suit et à l'examen des figures qui l'accompagnent, celles-ci ne sont données qu'à titre indicatif et nullement limitatif de l'invention. Les figures montrent :
- figure 1 : un mobile radiologique muni du dispositif anti-basculement de l'invention ;
- figure 2 : un exemple préféré de réalisation du mécanisme anti-basculement de l'invention ;
- figure 3 : des détails de réalisation du mécanisme de la figure 2 ;
- figure 4 : une représentation schématique des tolérances d'accès dans l'espace avec le tube à rayons X du mobile de l'invention.

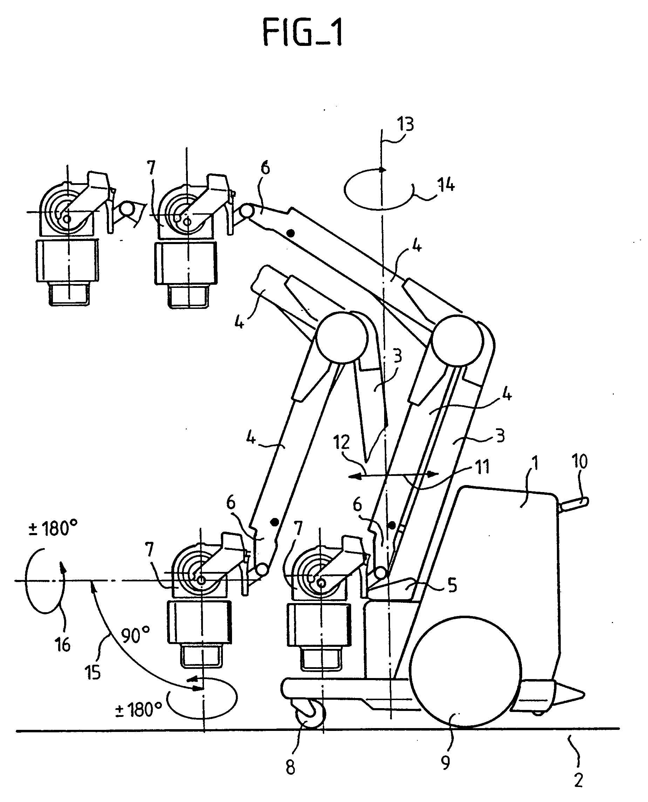

La figure 1 montre un mobile radiologique qui peut être muni du dispositif anti-basculement de l'invention. Ce mobile comporte essentiellement un chariot 1 mobile sur le sol 2 et portant une structure déployable 3, 4 représentée ici dans diverses positions de déploiement. La structure déployable est fixée par une première extrémité 5 sur le chariot et elle porte à sa deuxième extrémité 6 un tube à rayons X 7 ainsi que tous les moyens logistiques associés à ce tube et nécessaires pour le faire fonctionner. Le chariot comporte une partie avant munie de petites roulettes orientables 8 et une partie arrière portée de part et d'autre par deux grandes roues 9. Sur la figure on ne voit qu'un côté du mobile. Pour être déplacé le chariot comporte également une poignée de manoeuvre 10.

Hormis les déplacements du chariot sur le sol 2, la structure déployable peut être mobilisée de plusieurs façons. D'une part, elle peut subir un premier mouvement représenté par les flèches 11 et 12 par lequel sa structure se déploie en porte à faux vers l'arrière ou vers l'avant par rapport à l'assise du chariot 1. Ainsi, à l'exception de la position de garage dans laquelle le tube 7 est ramené à proximité de l'extrémité 5, dans les cas montrés l'aplomb du tube à rayons X est au-delà des roues avant 8. Il existe alors un couple de basculement naturel, vers l'avant, qui est d'autant plus grand que le porte à faux est plus grand. Cependant, comme tous les circuits de contrôle sont maintenus à l'arrière du chariot, leur poids exerce un couple naturel de réaction.

La structure déployable peut, par ailleurs, accepter un deuxième mouvement : un mouvement de rotation autour d'un axe vertical 13 montré par la flèche 14. Dans un exemple ce mouvement est limité mécaniquement, de part et d'autre de la direction principale du chariot mesurée selon sa longueur à ± 90°. On verra par la suite que compte-tenu du caractère rectangulaire du chariot, l'extension de déploiement n'est plus aussi importante sur les côtés que vers l'avant pour respecter les mêmes normes anti-basculement, compte-tenu du poids faible retenu pour le chariot. Enfin, le tube à rayons X 7 peut lui-même être animé de deux mouvements ; un premier mouvement 15 permettant de faire varier l'incidence et un deuxième mouvement 16 permettant d'orienter le champ de détection dans le cas où on prendrait un champ de détection de type rectangulaire. Ces deux derniers mouvements sont facultatifs. Ce qui compte est la mesure du porte à faux du tube 7 par rapport à l'embase du chariot 1.

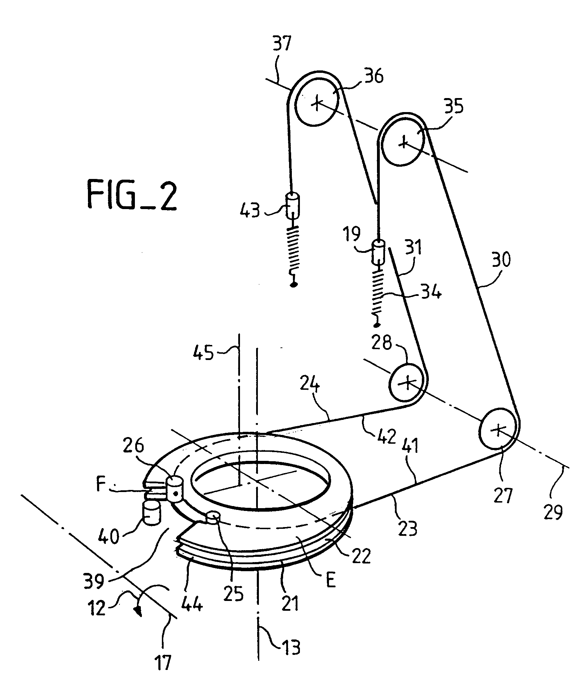

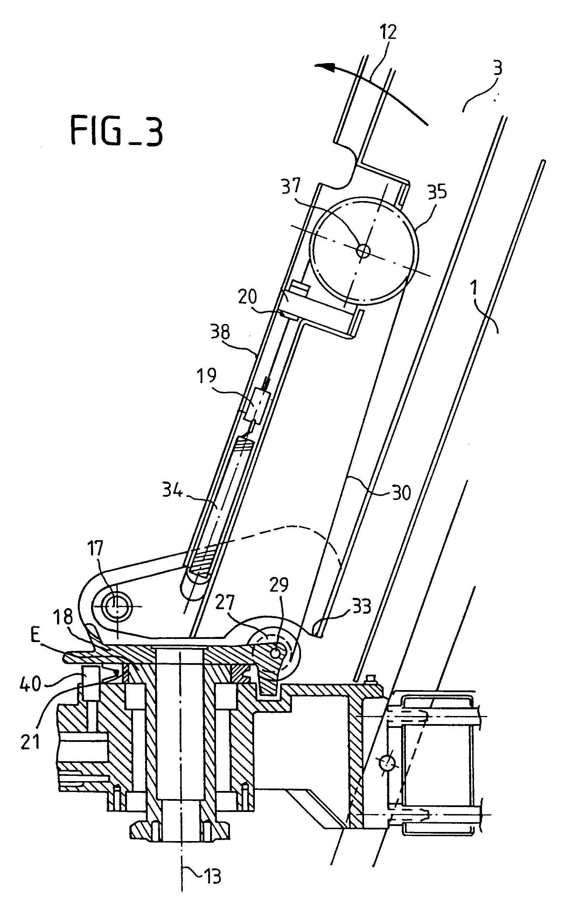

La figure 2 montre, en commun avec la figure 3, comment fonctionne d'une manière préférée le mécanisme anti-basculement de l'invention. L'extrémité 5 de la structure 3-4 est articulée sur un axe de rotation horizontal 17 perpendiculaire au plan de la figure 3 vue en coupe. En conséquence, le premier bras 3 de la structure peut basculer vers l'avant en tournant autour de l'axe 17. Au cours de ce basculement vers l'avant, son inclinaison change. Cette inclinaison est mesurée par le dispositif de l'invention, en même temps que la position en rotation de la base 18 autour de l'axe 13.

Dans l'exemple préféré de réalisation, la base 18 est posée sur une poulie folle 21 qui peut elle aussi tourner autour de l'axe 13. Cette poulie folle possède une gorge 22 à l'intérieur de laquelle est fixé, au moins en un endroit, un ou des câbles. Par exemple, deux câbles 23 et 24 sont partiellement enroulés dans la gorge et sont fixés par leurs extrémités, respectivement 25 et 26, de manière à ne pas pouvoir glisser au fond de cette gorge. Plutôt que d'avoir deux câbles, on peut avoir un câble continu qui serait à ce moment fixé en un seul endroit au fond de la gorge 22. Sur la figure 2 on a également montré l'axe 17 ainsi que le mouvement 12 de variation de l'inclinaison. L'axe 17 est orthogonal à l'axe 13 : il est placé légèrement vers l'avant du chariot, en direction des petites roues. Les câbles 23 et 24 passent par ailleurs sur des poulies de renvoi, respectivement 27 et 28, qui peuvent tourner autour d'un autre axe horizontal dit de renvoi 29. L'axe 29 est également porté par la base 18. Ceci est aussi visible sur la figure 3.

Lorsque le bras 3 est incliné vers l'avant, des brins 30 et 31 du câble 23 se déplacent à l'intérieur de ce bras 3. Ils s'y déplacent parce que le bras 3 pivote autour de l'axe 17, il en résulte donc que l'extrémité arrière 33 du bras 3 s'éloigne des poulies 27 et 28 et de l'axe de renvoi 29. Comme l'axe 29 est fixe horizontalement, les brins 30 et 31 descendent dans le bras 3 quand le bras 3 est incliné vers l'avant. Quand les brins 30 et 31 descendent, des butées mobiles telle que 19 remontent en direction de butées fixes telle que la butée.20. La butée 20 est fixée au bras 3. La butée 19 est fixée d'une part à l'extrémité du brin 30 du câble 23 et est fixée d'autre part à une extrémité d'un ressort 34 dont l'autre extrémité est par ailleurs fixée au bras 3 ou à la base 18. La butée 19 a ici l'allure d'un manchon serti sur le câble 23. L'endroit où est fixé la deuxième extrémité du ressort 34 n'est pas important, ce qui compte c'est que le câble 23 et les brins 30 et 31 soient maintenus d'une part au fond des gorges et d'autre part tendus dans le bras 3.

Le mouvement de glissement du bras 3, et des brins 30 et 31, dans le bras 3 provoque les remontées des butées mobiles 19 du fait de la présence, à titre de perfectionnement, de poulies dites de présentation, 35 et 36 tournant autour d'un axe dit de présentation horizontal 37. L'axe 37 est fixé au bras 3. La justification des poulies 35 et 36 se trouve dans le fait qu'on réalise dans le bras 3 une lumière 38 pour venir régler en hauteur la position de la butée fixe 20. Cette butée fixe 20 est par exemple creuse : le câble 23 passe à travers. Comme les longueurs des câbles 23 ou 24 peuvent varier en fonction de la fabrication, on remédie à l'inconvénient présenté par ces longueurs non constantes en réglant la hauteur de la butée 20. La butée 20 est, par exemple, munie à l'extérieur d'un filet : elle est plus ou moins vissée sur un support qui la maintient dans le bras 3. Si on n'avait pas réalisé la fenêtre 38 vers l'avant du bras 3, on n'aurait pas eu besoin des poulies 35 et 36. Mais dans ce cas la fenêtre 38 se serait trouvée vers l'arrière du bras 3 : à un endroit où on est susceptible de mettre les mains pour pousser manuellement le bras 3 vers l'avant. La présence de cette fenêtre aurait été alors dangereuse.

On a vu comment, lorsqu'on incline le bras 3 vers l'avant, la butée 19 vient au contact de la butée 20. Lorsqu'elle est au contact on ne peut plus incliner le bras 3 plus vers l'avant. On va montrer maintenant comment la hauteur de la butée 19 est réglable en fonction de la rotation de la structure déployable et donc du bras 3 autour de l'axe vertical 13. Quand cette structure tourne, la poulie folle 21 est entraînée par frottement : elle tourne avec la base 18. Ou bien elle est entraînée par réaction d'une des butées mobiles sur la butée fixe correspondante.

La poulie folle 21 est munie d'une encoche 39 à l'intérieur de laquelle est engagé un pivot 40. Le pivot 40 est solidaire du chariot 1. Le pivot 40 est fixe quand la structure tourne. En conséquence, quand un bord de l'encoche vient au contact du pivot 40, la poulie folle 21 ne peut plus tourner elle est arrêtée. A ce moment, si on continue à faire tourner la structure, la base 18 entraînant avec elle l'axe 29, déplace les poulies 27 et 28 dans un plan horizontal. Dans ces conditions, un brin 41 du câble 23, qui va de la poulie 27 à la poulie 21, s'enroule dans la gorge 22 de la poulie 21. De l'autre côté, un brin 42 du câble 24 se déroule de la poulie 21. Comme le câble 41 s'enroule, la butée 19 remonte (figure 2). Autrement dit, pour un mouvement dans le sens trigonométrique, la butée mobile 19 monte : c'est elle qui fixe, en fonction de la valeur de l'orientation, la hauteur de la limite d'inclinaison. Dans le cas de rotation dans le sens des aiguilles d'une montre c'est la hauteur de l'autre butée mobile 43 qui fixe la limite de l'inclinaison.

Autrement dit, si la structure est repliée et qu'on la fait tourner autour de l'axe 13 les butées mobiles 19 et 43 se calent en hauteur selon l'angle d'orientation de la structure. Si par la suite on veut incliner vers l'avant le bras 3 on en est empêché par la première de ces butées mobiles qui vient au contact d'une des butées fixes du bras 3.

Par contre, si on a déployé la structure entièrement vers l'avant, à orientation nulle, et qu'on fait tourner ensuite la structure autour de l'axe 13 la montée d'une des butées mobiles 19 ou 43 provoque un repliement automatique en fonction de l'angle de rotation.

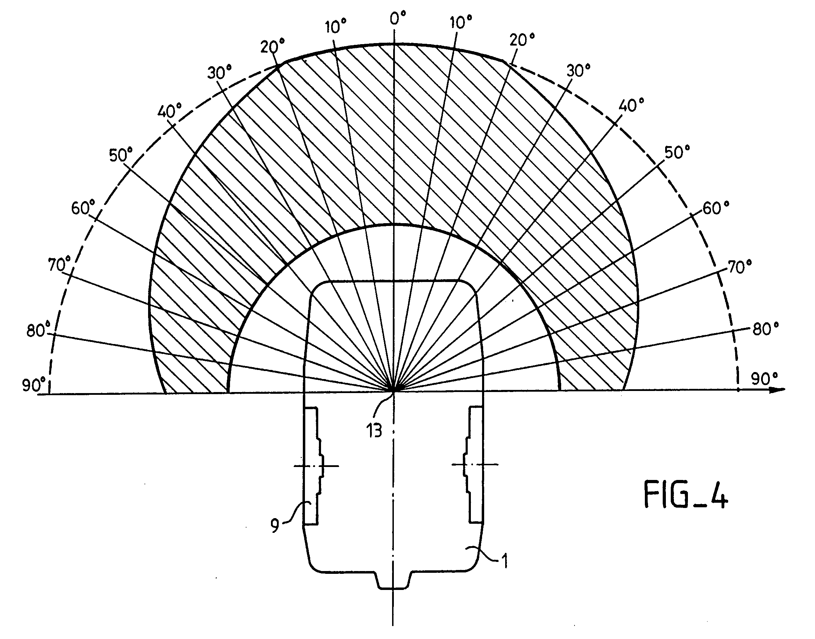

Ceci est montré d'une manière schématique sur la figure 4. On voit le chariot 1 ainsi que ses grandes roues 9. La structure n'est pas représentée mais on a indiqué dans la zone hachurée les positions tolérées de l'aplomb du tube à rayons X 7 par rapport à l'axe 13. Vers l'avant, lorsque l'orientation est 0°, le déploiement est maximal. Lorsque l'on arrive à des orientations de l'ordre de 20°, le déploiement est réduit progressivement : il ne suit plus l'arc de cercle montré en tirets mais il vient au contraire se réduire peu à peu.

Pour réaliser d'une manière simple le secteur autorisé du mouvement de la poulie folle 21, l'encoche 39 est faite de la manière suivante. Le fond de la gorge 22 est rond et est usiné en prenant comme centre de courbure l'axe 13 autour duquel la poulie doit tourner. Par contre, les sommets 44 de la gorge 22 sont, eux aussi ronds, mais centrés sur un axe vertical décalé par rapport à l'axe 13. Par exemple, un axe 45 est décalé de 1 cm de l'axe 13. Autrement dit, le bord extérieur des gorges est excentré par rapport au fond des gorges. Dans la partie excentrée la plus large il est ensuite possible de tailler une encoche 39. L'usinage est très simple. On aurait pu cependant remplacer l'encoche par tout système de taquets soudés sur la poulie ou même éventuellement réaliser le pivot 40 différemment.

## Revendications

1. Mobile radiologique comportant un chariot (1) mobile sur le sol (2), et une structure (3,4) déployable reliée (18) mécaniquement au chariot par une première extrémité (5) et à un tube à rayons X (7) par sa deuxième extrémité (6), la structure pouvant être déployée en porte-à-faux par rapport au chariot et pouvant tourner autour d'un axe vertical (13), caractérisé en ce qu'il comporte des moyens (19-43) pour que l'amplitude maximum du déploiement soit fonction (Fig 4) de l'angle de rotation de cette structure autour de l'axe vertical.

2. Mobile radiologique selon la revendication 1, caractérisé en ce que la structure comporte un premier bras (3) relié mécaniquement au chariot par une première extrémité et à une première extrémité d'un deuxième bras (4) par sa deuxième extrémité, ce deuxième bras étant relié par sa deuxième extrémité au tube à rayons X, le premier bras pouvant être incliné (17, 11,12) par rapport à la verticale et pouvant tourner autour de l'axe vertical (13), et des moyens (19-43) pour que l'angle maximum d'inclinaison du premier bras (3) soit fonction de l'angle de rotation de ce premier bras (3) autour de l'axe vertical (13).

3. Mobile selon la revendication 2, caractérisé en ce que les moyens (19-43) pour rendre l'inclinaison fonction de la rotation comportent un jeu de butées (19,43) mobiles dont le calage est fonction de la rotation, et qui viennent au contact de butées fixes (20) quand l'inclinaison doit être empêchée.

4. Mobile selon la revendication 2 ou la revendication 3, caractérisé en ce que les moyens (19-43) pour rendre l'inclinaison fonction de la rotation comportent une poulie folle (21) tournant autour de l'axe vertical (13) mais dont la rotation est limitée dans un secteur (39), un câble (23-24) passant dans la poulie (21), une partie (25) de ce câble étant reliée fixement à la poulie (21), au moins une extrémité de ce câble étant reliée à une butée mobile (19,43), la position de cette butée mobile (19,43) étant calée par l'enroulement du câble (41, 42) autour de la poulie folle (21).

5. Mobile selon l'une quelconque des revendications 2 ou 3, caractérisé en ce que les moyens (19-43) pour rendre l'inclinaison fonction de la rotation comportent une poulie folle (21) tournant autour de l'axe vertical (13) mais dont la rotation est limitée dans un secteur, deux câbles (23,24) passant chacun dans la poulie, une des extrémités de chaque câble étant reliée fixement à la poulie et l'autre extrémité de chaque câble étant reliée à une butée mobile (19,43), les positions de ces butées mobiles étant calées par l'enroulement des câbles (23,24) autour de la poulie folle.

6. Mobile selon la revendication 4 ou la revendication 5, caractérisé en ce que le premier bras est inclinable par basculement autour d'un axe horizontal d'inclinaison (17) monté sur une base (18) tournant autour de l'axe vertical (13), le ou les câbles passant dans des poulies de renvoi (27,28) qui tournent autour d'un axe horizontal de renvoi (29), l'axe de renvoi étant également monté sur la base tournante, ces deux axes horizontaux (17,29) étant placés de part et d'autre de l'axe vertical (13), les butées mobiles (19,43) se déplaçant par rapport au premier bras (3) lors de l'inclinaison de ce premier bras.

7. Mobile selon l'une quelconque des revendications 4 à 6, caractérisé en ce que le premier bras (3) comporte des butées fixes creuses (20) au travers desquelles passe le ou les câbles (23,24), les butées mobiles (19,43) solidaires des extrémités du ou des câbles étant déplacées au cours de la rotation, pour leur calage, par l'effet de deux ressorts (34) fixés en tension aux extrémités du ou des câbles (23,24).

8. Mobile selon l'une quelconque des revendications 4 à 7, caractérisé en ce que le premier bras (3) comporte des poulies de présentation (35, 36) pour renvoyer un dispositif de réglage des butées fixes (20) sur une des faces (38) du premier bras.

9. Mobile selon l'une quelconque des revendications 4 à 8, caractérisé en ce que, pour limiter la rotation de la poulie folle (21) dans un secteur, cette poulie folle (21) comporte une encoche (39) et en ce qu'un pivot (40) solidaire du chariot (1) est engagé dans cette encoche (39).

10. Mobile selon l'une quelconque des revendications 4 à 9, caractérisé en ce que la poulie folle (21) possède une gorge (22) dont le fond rond est centré sur un premier axe vertical (13) et dont les bords (44) supérieurs ronds sont centrés sur un deuxième axe vertical (45) décalé par rapport au premier axe vertical (13).

## Claims

1. Mobile radiography unit having a carriage (1) able to move on the ground (2), and an extending structure (3, 4) connected mechanically to the carriage at a first end (5) and to an X-ray tube (7) at its second end (6), the structure being able to be extended so as to overhang in relation to the carriage and being able to rotate about a vertical axis (13), characterised in that it includes means (19-43) to ensure that the maximum degree of extension is a function (Fig 4) of the angle of rotation of this structure about the vertical axis.

2. Mobile radiography unit according to Claim 1, characterised in that the structure has a first arm (3) connected mechanically to the carriage by means of a first end and to a first end of a second arm (4) by its second end, this second arm being connected at its second end to the X-ray tube, the first arm being able to be inclined (17, 11, 12) with respect to the vertical and able to rotate about the vertical axis (13), and means (19-43) to ensure that the maximum angle of inclination of the first arm (3) is a function of the angle of rotation of this first arm (3) about the vertical axis (13).

3. Mobile unit according to Claim 2, characterised in that the means (19-43) for making the inclination a function of the rotation include a set of movable stops (19, 43), the setting of which is a function of the rotation, and which come into contact with fixed stops when inclination must be prevented.

4. Mobile unit according to Claim 2 or Claim 3, characterised in that the means (19-43) for making the inclination a function of the rotation include a loose pulley (21) rotating about the vertical axis (13) but whose rotation is limited within a sector (39), a cable (23-24) passing round the pulley (21), a part (25) of this cable being fixed to the pulley (21), at least one end of this cable being connected to a movable stop (19, 43), and the position of this movable stop (19, 43) being set by the winding of the cable (41, 42) around the loose pulley (21).

5. Mobile unit according to either one of Claims 2 or 3, characterised in that the means (19-43) for making the inclination a function of the rotation include a loose pulley (21) rotating about the vertical axis (13) but whose rotation is limited within a sector, two cables (23, 24) each passing round the pulley, one end of each cable being fixed to the pulley and the other end of each cable being connected to a movable stop (19, 43), the positions of these movable stops being set by the winding of the cables (23, 24) around the loose pulley.

6. Mobile unit according to Claim 4 or Claim 5, characterised in that the first arm is inclinable by tilting about a horizontal axis of inclination (17) located on a base (18) rotating about the vertical axis (13), the cable or cables passing round return pulleys (27, 28) which rotate about a horizontal return axis (29), the return axis also being located on the rotating base, these two horizontal axes (17, 29) being positioned on either side of the vertical axis (13), the movable stops (19, 43) moving in relation to the first arm (3) when this first arm is inclined.

7. Mobile unit according to any one of Claims 4 to 6, characterised in that the first arm (3) has fixed hollow stops (20) through which pass the cable or cables (23, 24), the movable stops (19, 43) fixed to the ends of the cable or cables being moved in the course of the rotation, for their setting, through the effect of two springs (34) fixed under tension to the ends of the cable or cables (23, 24).

8. Mobile unit according to any one of Claims 4 to 7, characterised in that the first arm (3) has presentation pulleys (35, 36) for returning a device for adjusting the fixed stops (20) on one of the sides (38) of the first arm.

9. Mobile unit according to any one of Claims 4 to 8, characterised in that, in order to limit the rotation of the loose pulley (21) within a sector, this loose pulley (21) has a recess (39), and in that a pivot (40) fixed to the carriage (1) is engaged in this recess (39).

10. Mobile unit according to any one of Claims 4 to 9, characterised in that the loose pulley (21) has a groove (22) whose rounded bottom is centred on a first vertical axis (13) and whose rounded top edges (44) are centred on a second vertical axis (45) offset in relation to the first vertical axis (13).

## Patentansprüche

1. Mobiles Röntgengerät, mit einem Fahrgestell (1), das auf dem Boden (2) beweglich ist und mit einem entfaltbaren Aufbau (3, 4), dessen erstes Ende (5) mechanisch mit dem Fahrgestell verbunden ist (18) und dessen zweites Ende (6) mit einer Röntgenröhre (7) verbunden ist, wobei der Aufbau bezüglich des Fahrgestells in freitragender Weise entfaltbar ist und um die senkrechte Achse (13) verdrehbar ist, dadurch gekennzeichnet, daß es eine Anordnung (1) aufweist, damit die maximale Amplitude der Entfaltung eine Funktion (Fig. 4) des Drehwinkels des Aufbaus um die senkrechte Achse ist.

2. Mobiles Röntgengerät nach Anspruch 1, dadurch gekennzeichnet, daß der Aufbau einen ersten Arm (3) aufweist, der mechanisch mit dem Fahrgestell über ein erstes Ende verbunden ist und mit einem ersten Ende eines zweiten Armes (4) über sein zweites Ende verbunden ist, wobei der zweite Arm über sein zweites Ende mit der Röntgenröhre verbunden ist und der erste Arm bezüglich der Senkrechten geneigt werden kann (17, 11, 12) und um die senkrechte Achse (13) verschwenkt werden kann, sowie eine Anordnung (19 bis 43) aufweist, damit der maximale Neigungswinkel des ersten Armes (3) eine Funktion des Verdrehwinkels dieses ersten Armes (3) um die senkrechte Achse (13) ist.

3. Mobiles Röntgengerät nach Anspruch 2, dadurch gekennzeichnet, daß die Anordnung (19 bis 43), aufgrund derer die Neigung eine Funktion der Verdrehung ist, ein Paar beweglicher Anschläge (19, 43) aufweist, deren Verschiebung eine Funktion der Verdrehung ist und welche feststehende Anschläge (20) berühren, wenn die Neigung verhindert werden soll.

4. Mobiles Röntgengerät nach Anspruch 2 oder 3, dadurch gekennzeichnet, daß die Anordnung (19 bis 43), mittels derer die Neigung eine Funktion der Verdrehung ist, eine bewegliche Rolle (21) aufweist, welche sich um die senkrechte Achse (13) dreht, deren Verdrehung jedoch in einem Sektor (39) begrenzt ist, wobei ein Kabel (23, 24) die Rolle (21) durchsetzt, wobei ein Abschnitt (25) des Kabels mit der Rolle (21) fest verbunden ist, während wenigstens ein Ende des Kabels mit einem beweglichen Anschlag (19, 43) verbunden ist und die Position dieses beweglichen Anschlags (19, 43) durch die Aufwicklung des Kabels (41, 42) um die bewegliche Rolle herum verschiebbar ist.

5. Gerät nach einem der Ansprüche 2 oder 3, dadurch gekennzeichnet, daß die Anordnung (19 bis 43), mittels derer die Neigung eine Funktion der Verdrehung ist, eine bewegliche Rolle (21) aufweist, die sich um die senkrechte Achse (13) dreht, deren Verdrehung jedoch in einem Sektor begrenzt ist, wobei zwei Kabel (23, 34) jeweils die Rolle durchsetzen, wobei eines der Enden eines jeden Kabels fest mit der Rolle verbunden ist und das andere Ende eines jeden Kabels mit einem beweglichen Anschlag (19, 43) verbunden ist und die Positionen dieser beweglichen Anschläge durch das Aufwickeln der Kabel (23, 24) um die bewegliche Rolle herum verschiebbar sind.

6. Gerät nach Anspruch 4 oder Anspruch 5, dadurch gekennzeichnet, daß der erste Arm durch Verschwenkung um eine waagrechte Neigeachse (17) geneigt werden kann, welche in einem Grundteil (18) angeordnet ist, welches um die senkrechte Achse (13) verdrehbar ist, wobei das oder die Kabel Umlenkrollen (27, 28) durchsetzen, welche sich um eine waagrechte Umlenkachse (29) drehen, wobei die Umlenkachse gleichzeitig auch am sich drehenden Grundteil angeordnet ist und die beiden waagrechten Achsen (17, 29) beidseits der senkrechten Achse (13) angeordnet sind, während die beweglichen Anschläge (19, 43) bezüglich des ersten Armes (3) während der Neigung dieses ersten Armes verschiebbar sind.

7. Gerät nach einem der Ansprüche 4 bis 6, dadurch gekennzeichnet, daß der erste Arm (3) feststehende hohle Anschläge (20) aufweist, welche von dem oder den Kabeln (23, 24) durchsetzt werden, wobei die beweglichen Anschläge (19, 43), die mit den Enden des oder der Kabel fest verbunden sind, während der Verdrehung bewegt werden, im Hinblick auf ihre Verschiebung durch die Einwirkung zweier Federn (34), welche unter Aufrechterhaltung einer Spannung an den Enden des oder der Kabel (23, 24) befestigt sind.

8. Gerät nach einem der Ansprüche 4 bis 7, dadurch gekennzeichnet, daß der erste Arm (3) Anzeigerollen (35, 36) aufweist, zur Umlenkung einer Einstellvorrichtung für die feststehenden Anschläge (20) auf eine der Oberflächen (38) des ersten Armes.

9. Gerät nach einem der Ansprüche 4 bis 8, dadurch gekennzeichnet, daß zur Begrenzung der Verdrehung der beweglichen Rolle (21) in einem Sektor diese bewegliche Rolle (21) eine Aussparung (39) aufweist und daß ein Zapfen (40), welcher fest mit dem Fahrgestell (1) verbunden ist, in diese Aussparung (39) eingreift.

10. Gerät nach einem der Ansprüche 4 bis 9, dadurch gekennzeichnet, daß die bewegliche Rolle (21) eine Nut (22) aufweist, deren abgerundeter Boden zu einer ersten senkrechten Achse (13) zentriert ist und deren obere abgerundete Ränder (44) zu einer zweiten senkrechten Achse (45) zentriert sind, welche bezüglich der ersten senkrechten Achse (13) verschoben ist.
